# EUROPEAN PATENT APPLICATION

(11) **EP 4 417 265 A1**
(43) Date of publication of application: **21.08.2024**
(21) Application number: 22880798.8
(22) Date of filing: 29.09.2022
(51) Int. Cl.: A61Q 17/04, A61K 8/37, A61K 8/92

(54) **COSMETIC COMPOSITION**

(30) Priority: 13.10.2021 JP 2021168438
(71) Applicant: Shiseido Company, Ltd., Chuo-ku Tokyo 104-0061 (JP)
(72) Inventor: UJIMOTO, Kei, Tokyo 104-0061 (JP); SAKAE, Heini, Tokyo 104-0061 (JP)
(74) Representative: Cabinet Beau de Loménie
(86) International application number: PCT/JP2022/036521
(87) International publication number: WO 2023/063102

(57) **Abstract**

To provide a cosmetic composition having further improved ultraviolet absorption ability in the UVA and UVB regions.

The cosmetic composition is composed of components (i) and (ii) described below, and an oil content which is capable of at least partially dissolving these components:
(i) a first compound, which has a structure of formula (I) below: 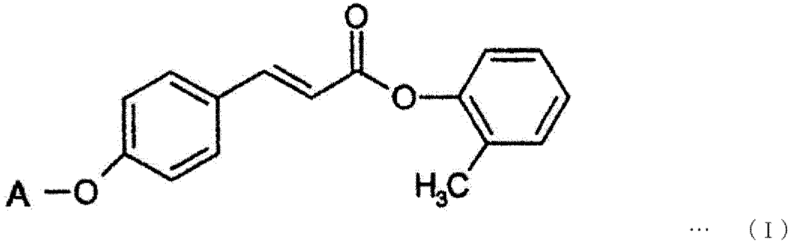 where -OA represents an alkoxy group, and
(ii) a second compound, which is different from the first compound, has a structure of any of (II-1), (II-2), (II-3), and (II-4) described below, and has a molecular weight of 250 or more: 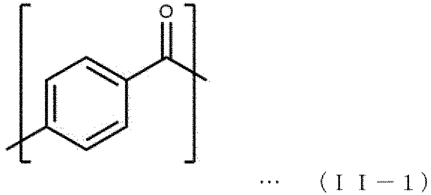 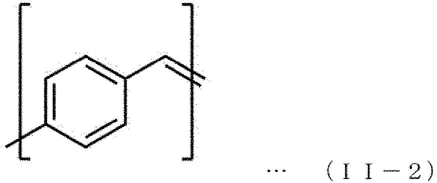 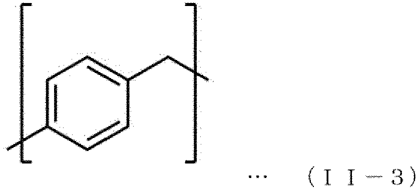 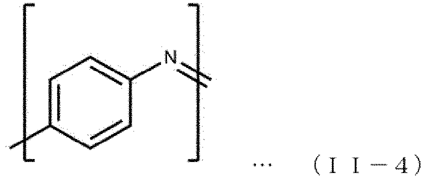 where the benzene ring may have a substituent.

## Description

### FIELD

The present invention relates to a cosmetic composition.

### BACKGROUND

Protecting the skin from ultraviolet rays is an important issue in skin care and body care. In addition to medium-wavelength ultraviolet rays (UVB region: wavelength 290 to 320 nm), which are known to cause sunburn and inflammation, long-wavelength ultraviolet rays (UVA region: wavelength 320 to 400 nm) are known to have an effect on the skin (photoaging, etc.).

Conventional cosmetics for ultraviolet protection often include 2-ethylhexyl p-methoxycinnamate as an ultraviolet absorber. Though 2-ethylhexyl p-methoxycinnamate has the effect of absorbing ultraviolet rays in the UVB region, there is a need for an ultraviolet absorber having better ultraviolet absorption ability in the UVA region.

In connection thereto, the ultraviolet absorber disclosed in Patent Literature 1 has absorption capability in both the UVA region and the UVB region, has an ultraviolet suppression effect in a wide range of wavelengths, and has a previously unseen property: the ultraviolet absorption capability thereof increases in the UVA and UVB regions with the passage of time of ultraviolet irradiation.

More specifically, the ultraviolet absorber of Patent Literature 1 contains, as an active ingredient, a compound represented by general formula I: 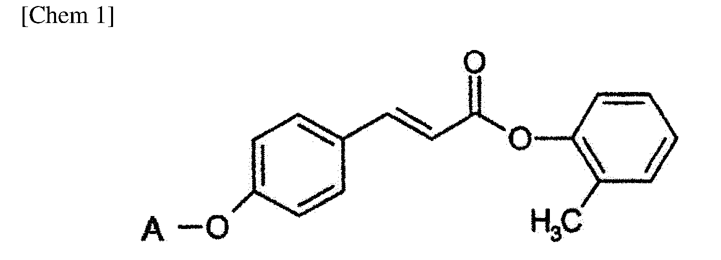 where -OA represents an alkoxy group.

### [CITATION LIST]

### [PATENT LITERATURE]

[PTL 1] WO 2009/041098

### SUMMARY

### [TECHNICAL PROBLEM]

In Patent Literature 1, the compound represented by general formula I above is included as-is into an ointment or into a liquid medium to exhibit ultraviolet absorption ability in the UVA and UVB regions.

However, the ultraviolet absorber of Patent Literature 1 still has room for improvement in terms of ultraviolet absorption ability in the UVA and UVB regions.

The present invention aims to improve the above situation, and an object thereof is to provide a cosmetic composition having further improved ultraviolet absorption ability in the UVA and UVB regions.

### [SOLUTION TO PROBLEM]

The present invention, which can achieve the object described above, is as described below.

### <Aspect 1>

A cosmetic composition, comprising components (i) and (ii) described below, and an oil content which is capable of at least partially dissolving these components:
(i) a first compound, which has a structure of formula (I) below: 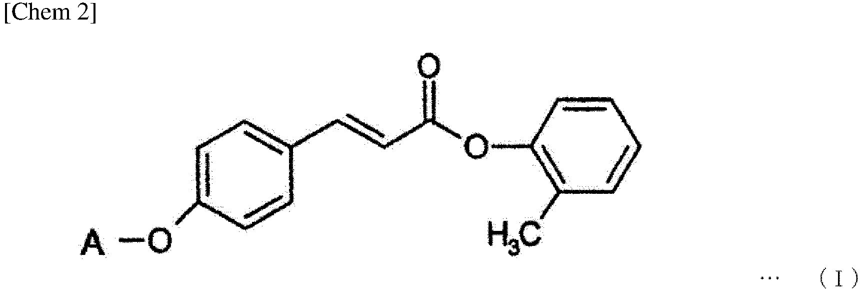 where -OA represents an alkoxy group, and
(ii) a second compound, which is different from the first compound, has a structure of any of (II-1), (II-2), (II-3), and (II-4) described below, and has a molecular weight of 250 or more: 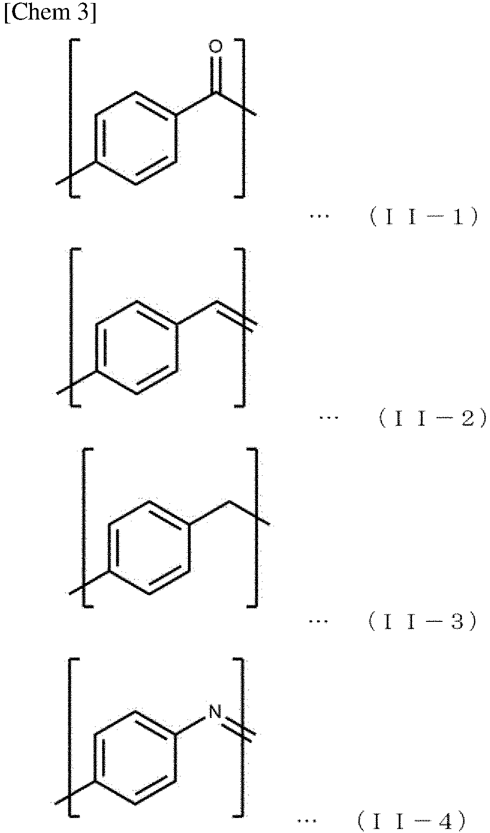 where the benzene ring may have a substituent.

### <Aspect 2>

The composition according to Aspect 1, wherein the second compound is at least one compound selected from the group consisting of ethylhexyl methoxycinnamate, octocrylene, ethylhexyl salicylate, homosalate, bisethylhexyloxyphenol methoxyphenyl triazine, diethylaminohydroxybenzoylhexyl benzoate, ethylhexyl triazone, polysilicone-15, t-butylmethoxydibenzoylmethane, phenylbenzimidazole sulfonic acid, and terephthalylidene dicamphor sulfonic acid.

### <Aspect 3>

The composition according to Aspect 1 or 2, wherein the oil content contains a polar oil.

### <Aspect 4>

The composition according to Aspect 3, wherein the first compound is 0.01 parts by mass or more and 50 parts by mass or less relative to a total of 100 parts by mass of the polar oil and the first compound.

### <Aspect 5>

The composition according to any one of Aspects 1 to 4, which is an oil-in-water cosmetic.

### <Aspect 6>

The composition according to any one of Aspects 1 to 4, which is a water-in-oil cosmetic.

### <Aspect 7>

The composition according to any one of Aspects 1 to 4, which is an oil-based cosmetic.

### <Aspect 8>

The composition according to any one of Aspects 1 to 7, which is a sunscreen cosmetic composition.

### [ADVANTAGEOUS EFFECTS OF INVENTION]

According to the present invention, there can be provided a cosmetic composition having further improved ultraviolet absorption ability in the UVA and UVB regions.

### DESCRIPTION OF EMBODIMENTS

The embodiments of the present invention will be described below. Note that the present invention is not limited to the following embodiments, and various changes can be made within the scope of the spirit of the invention.

### <<Cosmetic Composition>>

The cosmetic composition of the present invention (hereinafter also referred to simply as "the composition of the present invention") is:
a cosmetic composition, comprising components (i) and (ii) described below, and an oil content which is capable of at least partially dissolving these components:
(i) a first compound, which has a structure of formula (I) below: 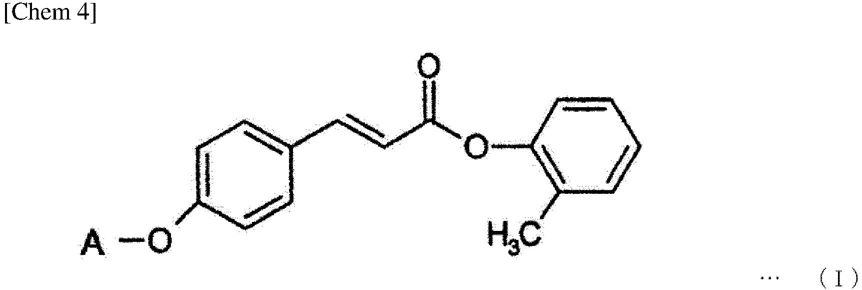 where -OA represents an alkoxy group, and
(ii) a second compound, which is different from the first compound, has a structure of any of (II-1), (II-2), (II-3), and (II-4) described below, and has a molecular weight of 250 or more.

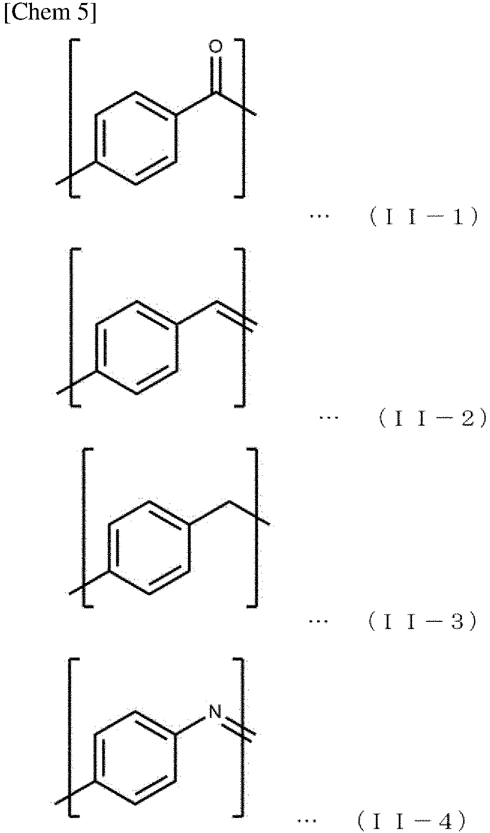

As a result of rigorous investigation by the present inventors, it was discovered that by using a first compound in combination with a second compound having a specific structure and specific molecular weight, the ultraviolet absorption ability in the UVA and UVB regions of a conventional cosmetic composition could be further improved.

More specifically, in the composition of the present invention, by using, in combination with a first compound having ultraviolet absorption ability in the UVA and UVB regions, a second compound having structural portions which are similar to the first compound and having a predetermined molecular weight (250 or more), a remarkable effect of improving the ultraviolet absorption effect can be obtained. Without being limited by theory, it is believed that by using a specific second compound, self-stacking of the first compound can be suppressed, whereby the ultraviolet absorption effect is further improved.

### <Component (i): First Compound>

In the present invention, the first compound has a structure of the following formula (I): 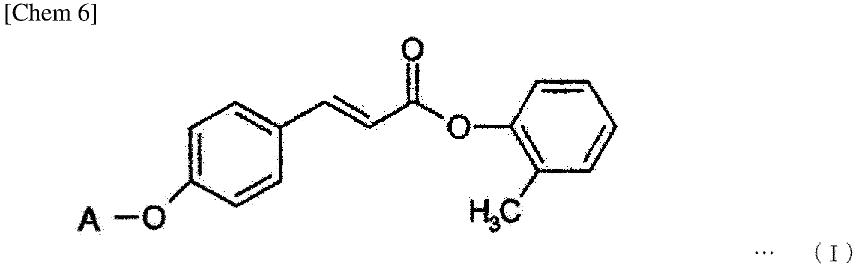 where -OA represents an alkoxy group.

In formula (I), -OA represents an alkoxy group, and more specific examples thereof include, but are not limited to, a methoxy group and an ethoxy group.

In the present invention, the first compound may have the same structure as the compound represented by general formula I disclosed in Patent Literature 1.

Furthermore, in the composition of the present invention, from the viewpoint of further exhibiting the effects of the present invention, it is preferable that the first compound be at least partially in a dissolved state, in particular 50% by mass or more thereof be in a dissolved state, more particularly 90% by mass or more thereof be in a dissolved state, further particularly 99% by mass or more thereof be in a dissolved state, and most particularly 100% by mass thereof be in a dissolved state.

In the composition of the present invention, the content of the first compound is not particularly limited, and may be, relative to the entire cosmetic composition, for example, 0.01% by mass or more, 0.05% by mass or more, 0.1% by mass or more, 0.5% by mass or more, 1.0% by mass or more, 1.5% by mass or more, 2.0% by mass or more, 2.5% by mass or more, 3.0% by mass or more, 3.5% by mass or more, 4.0% by mass or more, 4.5% by mass or more, 5.0% by mass or more, 5.5% by mass or more, 6.0% by mass or more, 6.5% by mass or more, 7.0% by mass or more, 7.5% by mass or more, 8.0% by mass or more, 8.5% by mass or more, 9.0% by mass or more, 9.5% by mass or more, or 10% by mass or more, and may be 50% by mass or less, 40% by mass or less, 30% by mass or less, 20% by mass or less, 10% by mass or less, 8.0% by mass or less, 5.0% by mass or less, or 2.0% by mass or less.

### <Component (ii): Second Compound>

In the present invention, the second compound is different from the first compound described above, has a structure of any of formulas (II-1), (II-2), (II-3), and (II-4) below, and has a molecular weight of 250 or more: 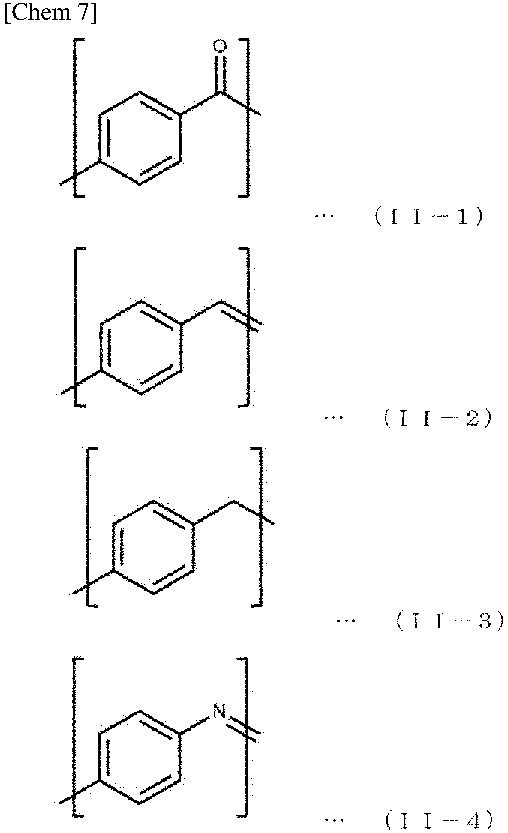 where the benzene ring may have a substituent.

In the present invention, as long as the second compound contains, in the structural formula thereof, a structure of any of formulas (II-1), (II-2), (II-3), and (II-4) above, the benzene ring of the structure of any of formulas (II-1), (II-2), (II-3), and (II-4) may further have one or more substituents.

In the present invention, examples of the substituent include, but are not limited thereto, an alkyl group or an alkyl group that may be further substituted with another substituent, a cycloalkyl group or a cycloalkyl group that may be further substituted with another substituent, an alkenyl group or an alkenyl group that may be further substituted with another substituent, an alkynyl group or an alkynyl group that may be further substituted with another substituent, an aryl group or an aryl group which may be further substituted with another substituent, a carbonyl group, a hydroxyl group, an alkoxyl group, an amino group or an amino group that may be further substituted with another substituent, a heterocyclic group or a heterocyclic group that may be further substituted with another substituent, a sulfonic acid group, and a halogen atom.

More specifically, the molecular weight of the second compound may be, for example, 250 or more, 260 or more, 270 or more, 280 or more, 290 or more, 300 or more, 310 or more, 320 or more, 330 or more, 340 or more, 350 or more, 360 or more, 370 or more, 380 or more, 390 or more, 400 or more, 450 or more, 500 or more, 550 or more, 600 or more, 650 or more, 700 or more, 750 or more, or 800 or more, and the upper limit thereof is not particularly limited, and may be, for example, 10,000 or less, 8,000 or less, 7,000 or less, 6,000 or less, 5,000 or less, 4,000 or less, 3,000 or less, 2,000 or less, or 1,000 or less.

In the present invention, the second compound may be, but is not limited to, at least one compound selected from the group consisting of ethylhexyl methoxycinnamate (molecular weight: 290), octocrylene (molecular weight: 361), ethylhexyl salicylate (molecular weight: 250), homosalate (molecular weight: 262), bisethylhexyloxyphenol methoxyphenyl triazine (molecular weight: 628), diethylaminohydroxybenzoylhexyl benzoate (molecular weight: 398), ethylhexyl triazone (molecular weight: 823), polysilicone-15 (molecular weight: approximately 6000), t-butylmethoxydibenzoylmethane (molecular weight: 310), phenylbenzimidazole sulfonic acid (molecular weight: 274), terephthalylidene dicamphor sulfonic acid (molecular weight: 563), 2-ethylhexyl dimethoxybenzylidene dioxoimidazolidine propionate (molecular weight: 435), methylbis(trimethylsiloxy)silylisopentyl trimethoxycinnamate (molecular weight: 529), and methylenebisbenzotriazolyltetramethylbutylphenol (molecular weight: 659). Among these, at least one compound selected from the group consisting of ethylhexyl salicylate (molecular weight: 250), bisethylhexyloxyphenol methoxyphenyl triazine (molecular weight: 628), ethylhexyl triazone (molecular weight: 823), polysilicone-15 (molecular weight: approximately 6000), t-butylmethoxydibenzoylmethane (molecular weight: 310), phenylbenzimidazole sulfonic acid (molecular weight: 274), and terephthalylidene dicamphor sulfonic acid (molecular weight: 563) is preferable, and at least one compound selected from the group consisting of polysilicone-15 (molecular weight: approximately 6000), phenylbenzimidazole sulfonic acid (molecular weight: 274), and terephthalylidene dicamphor sulfonic acid (molecular weight: 563) is more preferable.

Furthermore, in the composition of the present invention, from the viewpoint of further exhibiting the effects of the present invention, it is preferable that the second compound in the case of a solid be at least partially in a dissolved state, in particular 50% by mass or more thereof be in a dissolved state, more particularly 90% by mass or more thereof be in a dissolved state, further particularly 99% by mass or more thereof be in a dissolved state, and most particularly 100% by mass thereof be in a dissolved state.

In the composition of the present invention, the content of the second compound is not particularly limited, and may be, for example, relative to the entire cosmetic composition, 0.01% by mass or more, 0.05% by mass or more, 0.1% by mass or more, 0.5% by mass or more, 1.0% by mass or more, 1.5% by mass or more, 2.0% by mass or more, 2.5% by mass or more, 3.0% by mass or more, 3.5% by mass or more, 4.0% by mass or more, 4.5% by mass or more, 5.0% by mass or more, 5.5% by mass or more, 6.0% by mass or more, 6.5% by mass or more, 7.0% by mass or more, 7.5% by mass or more, 8.0% by mass or more, 8.5% by mass or more, 9.0% by mass or more, 9.5% by mass or more, or 10% by mass or more, and may be 50% by mass or less, 40% by mass or less, 30% by mass or less, 20% by mass or less, 10% by mass or less, 8.0% by mass or less, 5.0% by mass or less, or 2.0% by mass or less.

### <Oil Content>

The composition of the present invention contains an oil content. The oil content contained in the composition of the present invention is an oil content that can at least partially dissolve the first compound and the second compound, and thus, these components (i.e., the first compound and the second compound) can be at least partially dissolved therein.

In the composition of the present invention, the content of the oil content is not particularly limited, and may be, for example, 5.0% by mass or more, 10% by mass or more, 15% by mass or more, 20% by mass or more, 25% by mass or more, 30% by mass or more, 35% by mass or more, 40% by mass or more, or 50% by mass or more, and may be 99.9% by mass or less, 90% by mass or less, 80% by mass or less, 70% by mass or less, 60% by mass or less, 55% by mass or less, 50% by mass or less, 45% by mass or less, 40% by mass or less, 35% by mass or less, or 30% by mass or less.

In the present invention, the oil content is not particularly limited as long as the first compound and second compound described above can be at least partially dissolved therein. More specifically, the oil content may contains one or more of, for example, a polar oil, a silicone oil, or a hydrocarbon oil. From the viewpoint of dissolving the first compound described above, the oil content preferably contains a polar oil.

### (Polar Oil)

"Polar oil" as used herein refers to polar oils with high polarity among oils usable in cosmetics, other than silicone oils, and refers to those having an IOB value of, for example, 0.10 or more, 0.11 or more, 0.12 or more, or 0.13 or more. Furthermore, the IOB value of the polar oil according to the present invention may be 0.50 or less, 0.45 or less, or 0.40 or less. The IOB value is an abbreviation of Inorganic/Organic Balance, is a value representing the ratio of the inorganic value to the organic value, and is an index indicating the degree of polarity of the organic compound. Specifically, the IOB value is expressed as IOB value = inorganic value / organic value. Regarding the "inorganic value" and the "organic value," depending on the type of atom or functional group, the "inorganic value" or "organic value" is set in a molecule, for example, one carbon atom has an "organic value" of 20 and one hydroxyl group has an "inorganic value" of 100, and by integrating the "inorganic value" or "organic value" of each atom and functional group in an organic compound, the IOB value of the organic compound can be calculated (for example, refer to Yoshio KODA, "Organic Conceptual Diagram - Basics and Applications," pp. 11-17, published by Sankyo Publishing, 1984).

Note that in the present invention, first compounds and second compounds having an IOB value of 0.10 or more are excluded from the "polar oils" herein.

In the present invention, the polar oil may be, for example, at least one selected from the group consisting of ester oils, ether oils, higher alcohols, and fatty acids having an IOB value of 0.10 or more.

More specifically, the polar oil may be, for example, at least one selected from the group consisting of bisethoxydiglycol cyclohexane-1,4-dicarboxylate (IOB value 1.03), phenoxyethyl caprylate (IOB value 0.29), PPG-30-BUTETH-30 (IOB value 0.94), diisopropyl sebacate (IOB value 0.4), neopentyl glycol diheptanoate (IOB value 0.33), diethylhexyl succinate (IOB value 0.32), isononyl isononanoate (IOB value 0.2), isopropyl myristate (IOB value = 0.18), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), and tri(caprylic acid/capric acid) glyceryl (IOB value = 0.33), but it is not limited thereto.

In the present invention, the amount of the polar oil contained in the oil content is not particularly limited, and may be, relative to a total of 100 parts by mass of the oil content, for example, 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less.

Moreover, when the oil content of the composition of the present invention contains a polar oil, the polar oil and the first compound described above may be included at the following composition ratio. More specifically, relative to a total of 100 parts by mass of the polar oil and the first compound, the first compound may be 0.01 parts by mass or more, 0.05 parts by mass or more, 0.1 parts by mass or more, 0.5 parts by mass or more, 1.0 part by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, 9.5 parts by mass or more, 10 parts by mass or more, 15 parts by mass or more, or 20 parts by mass or more, and may be 50 parts by mass or less, 45 parts by mass or less, 40 parts by mass or less, 35 parts by mass or less, 30 parts by mass or less, 25 parts by mass or less, 20 parts by mass or less, 15 parts by mass or less, or 10 parts by mass or less..

### (Silicone Oil)

In the present invention, "silicone oil" refers to oil contents having a main skeleton formed by siloxane bonds among those usable in cosmetics. In the present invention, second compounds which have a main skeleton formed by siloxane bonds are excluded from the "silicone oil" herein.

In the present invention, the silicone oil may be a volatile silicone oil or a nonvolatile silicone oil.

In the present invention, the boiling point under one atmosphere (101.325 kPa) can be used as a guideline for "volatile." The boiling point may be, for example, 250 °C or lower, 240 °C or lower, or 230 °C or lower, and may be 80 °C or higher, 100 °C or higher, 120 °C or higher, 150 °C or higher, or 160 °C or higher. Furthermore, in the present disclosure, "nonvolatile" is intended to mean that the volatile content is 5% or less when a sample is placed in a sufficiently large flat-bottomed dish and allowed to stand at 105 °C for 3 hours.

Furthermore, in the present invention, the silicone oil may be an acyclic silicone oil (i.e., a linear silicone oil) or a cyclic silicone oil.

Specific examples of the silicone oil include, but are not limited to, acyclic silicone oils such as polydimethylsiloxane (dimethicone), trisiloxane, caprylylmethicone, methylphenylpolysiloxane, and methylhydrogenpolysiloxane, cyclic silicone oils such as octamethylcyclotetrasiloxane, decamethylcyclopentasiloxane, and dodecamethylcyclohexasiloxane, and mixtures of two or more thereof. From the viewpoint of feeling during use, acyclic silicone is particularly preferable as the silicone oil.

In the present invention, the amount of the silicone oil contained in the oil content is not particularly limited, and may be, relative to a total of 100 parts by mass of the oil content, for example, 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less.

### (Hydrocarbon Oil)

In the present invention, "hydrocarbon oil" refers to hydrocarbon oils other than the polar oils described above.

The hydrocarbon oil may be a volatile hydrocarbon oil or a nonvolatile hydrocarbon oil.

Specific examples of the hydrocarbon oil include, but are not limited to, decane, dodecane, isododecane, isohexadecane, liquid paraffin, squalane, squalene, paraffin, and mixtures of two or more thereof.

### <Applications and Forms of Composition of Present Invention>

The composition of the present invention can be suitably used as a cosmetic or a raw material thereof. Thus, in particular, the present invention provides a sunscreen cosmetic composition.

The form of the composition of the present invention is not particularly limited, and may be, for example, an oil-in-water emulsified cosmetic composition, a water-in-oil emulsified cosmetic composition, or an oil-based cosmetic composition.

### <Other Components>

The cosmetic composition of the present invention may further contain, in addition to the above-mentioned components, arbitrary aqueous components or other components that can be used in the cosmetics field. As the other components, for example, other components such as ultraviolet scattering particles, surfactants, moisturizers, sequestering agents, natural and synthetic polymers, water-soluble and oil-soluble polymers, various extracts, colorants such as organic dyes, preservatives, antioxidants, pigments, thickeners, pH adjusters, fragrances, cooling agents, antiperspirants, disinfectants, skin activators, other chemicals, and various powders may be further contained, but the other components are not limited thereto.

### (Ultraviolet Scattering Agent Particles)

The composition of the present invention may further contain ultraviolet scattering agent particles from the viewpoint of enhancing the ultraviolet protection effect.

The ultraviolet scattering agent particles are not particularly limited, and may be, for example, particles of at least one metal oxide selected from the group consisting of titanium oxide, zinc oxide, iron oxide, and cerium oxide.

The ultraviolet scattering agent particles may be subjected to a hydrophobization treatment. The hydrophobization treatment may be performed using a known surface treatment agent for hydrophobization, and examples thereof include a fatty acid treatment, fluorine compound treatment, silicone treatment, silicone resin treatment, pendant treatment, silane coupling agent treatment, titanium coupling agent treatment, oil treatment, N-acylated lysine treatment, polyacrylic acid treatment, metal soap treatment, amino acid treatment, inorganic compound treatment, plasma treatment, mechanochemical treatment, silane compound treatment, and silazane compound treatment.

The average primary particle diameter of the ultraviolet scattering agent particles is not particularly limited, and may be, for example, 5 nm or more, 10 nm or more, or 15 nm or more, and may be 200 nm or less, 100 nm or less, or 50 nm or less. Note that the "average primary particle diameter" may be determined as the circle equivalent diameter of the projected area of the primary particles in an SEM image.

The shape of the ultraviolet scattering agent particles is not particularly limited, and examples thereof include spherical, plate-like, rod-like, spindle-like, needle-like, and irregular shapes.

In the composition of the present invention, when ultraviolet scattering agent particles are contained, the content thereof is not particularly limited, and is, for example, 1.0% by mass or more and 15% by mass or less.

### (Surfactant)

In the present invention, the surfactant may function as a dispersant or an emulsifier, or may have both of these functions.

The surfactant is not particularly limited, and examples thereof include, but are not limited to, isostearic acid, PEG-10 dimethicone, PEG-9 polydimethylsiloxyethyl dimethicone, and lauryl PEG-9 polydimethylsiloxyethyl dimethicone.

In the composition of the present invention, when a surfactant is contained, the content thereof is not particularly limited, and is, for example, 1.0% by mass or more and 10% by mass or less.

### (Water-Soluble Component)

The composition of the present invention may further contain a water-soluble component such as a lower alcohol. Examples of the lower alcohol include, but are not limited to, ethanol, propanol, butanol, pentanol, and hexanol.

Furthermore, when a water-soluble component is contained, the content thereof is not particularly limited, and may be, for example, 1.0 to 10% by mass.

### EXAMPLES

The present invention will be described in more detail below with reference to the Examples, but the present invention is not limited thereto.

### <<Preparation of Water-in-Oil Cosmetic Compositions

### <Example 1, Comparative Examples 1-1 and 1-2>

The compositions of Example 1 and Comparative Examples 1-1 and 1-2 were prepared based on the compositions (% by mass) of Table 1 below.

Note that in Example 1 and Examples 2 to 11, which are described later, compound I represented by the following structural formula was used as the first compound:

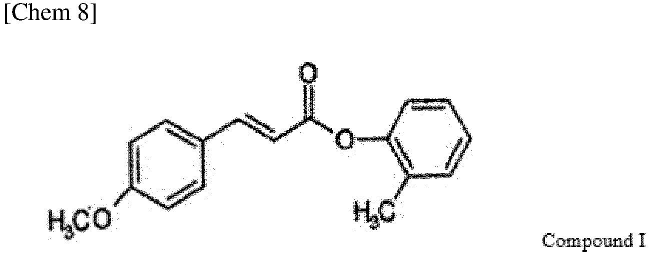

### (Evaluation)

The ultraviolet absorption wavelength of each prepared composition was measured, and the integrated value of absorbance at 290 to 400 nm was determined. The ultraviolet absorption ability improvement effect of each of the compositions of Example 1 and Comparative Example 1-2 relative to Comparative Example 1-1 (reference) was determined as the "boost rate." Specifically, the "boost rate" was calculated using the following formula: Boost rate = (integrated value of absorbance of Example 1 or Comparative Example 1-2 / integrated value of absorbance of Comparative Example 1-1) × 100%

Results of the obtained boost rates are shown in Table 1.

### [Table 1]

**(Table 1)**

| Composition | Comparative Example 1-1 | Example 1 | Comparative Example 1-2 |
|---|---|---|---|
| Water | 33.2 | 32.2 | 32.2 |
| Ethanol | 5.0 | 5.0 | 5.0 |
| Glycerin | 1.0 | 1.0 | 1.0 |
| Disteardimonium hectorite | 0.5 | 0.5 | 0.5 |
| PEG-9 polydimethylsiloxyethyl dimethicone | 2.0 | 2.0 | 2.0 |
| Isostearic acid | 0.5 | 0.5 | 0.5 |
| Diisopropyl sebacate (oil content) | 10 | 10 | 10 |
| Triethylhexanoin (oil content) | 10 | 10 | 10 |
| Isododecane (oil content) | 10 | 10 | 10 |
| Dimethicone (oil content) | 10 | 10 | 10 |
| Compound I (first compound) | - | 1.0 | - |
| Ethylhexyl methoxycinnamate (molecular weight: 290) (second compound) | 5.0 | 5.0 | 5.0 |
| Octocrylene (molecular weight: 361) (second compound) | - | - | 1.0 |
| Aluminum stearate-treated titanium oxide | 2.0 | 2.0 | 2.0 |
| Triethoxycaprylylsilane-treated zinc oxide | 5.0 | 5.0 | 5.0 |
| Silica powder | 5.0 | 5.0 | 5.0 |
| Phenoxyethanol | 0.5 | 0.5 | 0.5 |
| Sodium chloride | 0.1 | 0.1 | 0.1 |
| EDTA-3Na | 0.2 | 0.2 | 0.2 |
| Total | 100 | 100 | 100 |
| Boost rate | Reference | 138% | 115% |

As is clear from Table 1, it was found that the composition of Example 1, which contained the first and second compounds, had greatly improved ultraviolet absorption ability in the UVA and UVB regions as compared to Comparative Example 1-1, which did not contain the first compound.

Furthermore, it was found that though the composition of Comparative Example 1-2, which did not contain the first compound but which contained two second compounds, had a slightly improved ultraviolet absorption ability as compared to Comparative Example 1-1, the improvement in ultraviolet absorption ability was not as great as that of Example 1.

### <Examples 2 to 11 and Comparative Examples 2 to 13>

The compositions of Examples 2 to 11 and Comparative Examples 2 to 13 were prepared based on the compositions (% by mass) of Tables 2 and 3 below. The compositions of Comparative Examples 3 to 12, which did not contain the "first compound" and were compensated with an increased amount of water, were otherwise the same as the compositions of Examples 2 to 11, respectively. Furthermore, the composition of Comparative Example 13, which did not contain the "first compound" and was compensated with an increased amount of water, was otherwise the same as the composition of Comparative Example 2.

### (Evaluation)

In the same manner as Example 1 above, the integrated value of absorbance at 290 to 400 nm of each prepared composition was determined, and the ultraviolet absorption ability improvement effect of each of the compositions of Examples 2 to 11 and Comparative Example 2 was determined as the "boost rate." The results are shown in Table 2.

Note that when calculating the boost rate, Comparative Examples 3 to 13 were used as references for Examples 2 to 11 and Comparative Example 2, respectively.

**[Table 2]**

| (Table 2: Example 2 to 11 and Comparative Example 2) | | | | | | | | | | | | |
|---|---|---|---|---|---|---|---|---|---|---|---|---|
| Composition | | Example 2 | Example 3 | Example 4 | Example 5 | Example 6 | Example 7 | Example 8 | Example 9 | Example 10 | Example 11 | Comparati ve Example 2 |
| Water | Water | 32.2 | 32.2 | 32.2 | 34.2 | 34.2 | 35.2 | 32.2 | 34.2 | 35.2 | 35.2 | 35.2 |
| First compound | Compound I | 1.0 | 1.0 | 1.0 | 1.0 | 1.0 | 1.0 | 1.0 | 1.0 | 1.0 | 1.0 | 1.0 |
| | Octocrylene (molecular weight: 361) | 5.0 | - | - | - | - | - | - | - | - | - | - |
| | Ethylhexyl salicylate (molecular weight: 250) | - | 5.0 | - | - | - | - | - | - | - | - | - |
| | Homosalate (molecular weight: 262) | - | - | 5.0 | - | - | - | - | - | - | - | - |
| | Bisethylhexyloxyphenolmethoxyphenyltriazine (molecular weight: 628) | - | - | - | 3.0 | - | - | - | - | - | - | - |
| Second compound | Diethylaminohydroxybenzoylhexyl benzoate (molecular weight: 398) | - | - | - | - | 3.0 | - | - | - | - | - | - |
| | Ethylhexyltriazone (molecular weight: 823) | - | - | - | - | - | 2.0 | - | - | - | - | - |
| | Polysilicone-15 (molecular weight: approx. 6000) | - | - | - | - | - | - | 5.0 | - | - | - | - |
| | t-butylmethoxydibenzoylmethane (molecular weight: 310) | - | - | - | - | - | - | - | 3.0 | - | - | - |
| | Phenylbenzimidazole sulfonic acid (molecular weight: 274) | - | - | - | - | - | - | - | - | 2.0 | - | - |
| | Terephthalylidene dicamphor sulfonic acid (molecular weight: 563) | - | - | - | - | - | - | - | - | - | 2.0 | - |
| Third compound | Oxybenzone-3 (molecular weight: 228) | - | - | - | - | - | - | - | - | - | - | 2.0 |
| Other | Other | 61.8 | 61.8 | 61.8 | 61.8 | 61.8 | 61.8 | 61.8 | 61.8 | 61.8 | 61.8 | 61.8 |
| Total | | 100 | 100 | 100 | 100 | 100 | 100 | 100 | 100 | 100 | 100 | 100 |
| Evaluation | Boost rate | 145% | 161% | 142% | 160% | 158% | 164% | 167% | 161% | 168% | 167% | 110% |
| | Boost rate calculation reference | Comp Ex 3 | Comp Ex 4 | Comp Ex 5 | Comp Ex 6 | Comp Ex 7 | Comp Ex 8 | Comp Ex 9 | Comp Ex 10 | Comp Ex 11 | Comp Ex 12 | Comp Ex 13 |

**[Table 3]**

| (Table 3: Comp Ex 3 to 13) | | | | | | | | | | | | |
|---|---|---|---|---|---|---|---|---|---|---|---|---|
| Composition | | Comparati ve Example 3 | Comparati ve Example 4 | Comparati ve Eamplex 5 | Comparati ve Example 6 | Comparati ve Example 7 | Comparati ve Example 8 | Comparati ve Example 9 | Comparati ve Example 10 | Comparati ve Example 11 | Comparati ve Example 12 | Comparati ve Example 13 |
| Water | Water | 33.2 | 33.2 | 33.2 | 35.2 | 35.2 | 36.2 | 33.2 | 35.2 | 36.2 | 36.2 | 36.2 |
| First compound | Compound I | - | - | - | - | - | - | - | - | - | - | - |
| Second compound | Octocrylene (molecular weight: 361) | 5.0 | - | - | - | - | - | - | - | - | - | - |
| | Ethylhexyl salicylate (molecular weight: 250) | - | 5.0 | - | - | - | - | - | - | - | - | - |
| | Homosalate (molecular weight: 262) | - | - | 5.0 | - | - | - | - | - | - | - | - |
| | Bisethylhexyloxyphenolmethoxyphenyltriazine (molecular weight: 628) | - | - | - | 3.0 | - | - | - | - | - | - | - |
| | Diethylaminohydroxybenzoylhexyl benzoate (molecular weight: 398) | - | - | - | - | 3.0 | - | - | - | - | - | - |
| | Ethylhexyltriazone (molecular weight: 823) | - | - | - | - | - | 2.0 | - | - | - | - | - |
| | Polysilicone-15 (molecular weight: approx. 6000) | - | - | - | - | - | - | 5.0 | - | - | - | - |
| | t-butylmethoxydibenzoylmethane (molecular weight: 310) | - | - | - | - | - | - | - | 3.0 | - | - | - |
| | Phenylbenzimidazole sulfonic acid (molecular weight: 274) | - | - | - | - | - | - | - | - | 2.0 | - | - |
| | Terephthalylidene dicamphor sulfonic acid (molecular weight: 563) | - | - | - | - | - | - | - | - | - | 2.0 | - |
| Third compound | Oxybenzone-3 (molecular weight: 228) | - | - | - | - | - | - | - | - | - | - | 2.0 |
| Other | Other | 61.8 | 61.8 | 61.8 | 61.8 | 61.8 | 61.8 | 61.8 | 61.8 | 61.8 | 61.8 | 61.8 |
| Total | | 100 | 100 | 100 | 100 | 100 | 100 | 100 | 100 | 100 | 100 | 100 |

The details of "other" components of Tables 2 and 3 are as follows:

| | |
|---|---|
| ·Ethanol | 5.0% by mass |
| ·Glycerin | 1.0% by mass |
| ·Disteardimonium hectorite | 0.5% by mass |
| ·PEG-9 polydimethylsiloxyethyl dimethicone | 2.0% by mass |
| ·Isostearic acid | 0.5% by mass |
| ·Diisopropyl sebacate | 10% by mass |
| ·Triethylhexanoin | 10% by mass |
| ·Isododecane | 10% by mass |
| ·Dimethicone | 10% by mass |
| Aluminum stearate-treated zinc oxide | 2.0% by mass |
| ·Triethoxycaprylylsilane-treated zinc oxide | 5.0% by mass |
| ·Silica powder | 5.0% by mass |
| ·Phenoxyethanol | 5.0% by mass |
| ·Na chloride | 0.1% by mass |
| ·EDTA-3Na | 0.2% by mass |

As is clear from Table 2, Examples 2 to 11, which contained the first and second compounds, all had large boost rates (i.e., the ultraviolet absorption ability in the UVA and UVB regions was greatly improved) as compared to Comparative Examples 3 to 12, which contained, among the first to third compounds, only the second compound. From this, it is understood that the combination of the first compound and the second compound produces a synergistic effect.

Comparative Example 2, which contained the first and third compounds, had a small boost rate (i.e., there was no significant improvement in ultraviolet absorption ability in the UVA and UVB regions) as compared to Comparative Example 13, which contained, among the first to third compounds, only the third compound. From this, it is understood that no synergistic effect is produced by the combination of the first compound and the third compound.

### <Reference Examples 1 and 2>

The compositions of Reference Example 1, which contained the first compound but did not contain the second compound, and Reference Example 2, which contained the second compound but did not contain the first compound, were prepared based on the compositions of Table 4 below. Note that the "first compound" of Reference Example 1 was the same compound I as in Example 1 described above.

### (Evaluation)

The integrated value of absorbance at 290 to 400 nm of each prepared composition was determined, and the "boost rate" of one of the compositions was determined using the other as a reference. The results are shown in Table 4.

**[Table 4]**

| (Table 4: Reference Example 1 and 2) | | | |
|---|---|---|---|
| Composition | | Reference Example 1 | Reference Example 2 |
| Water | Water | 37.2 | 37.2 |
| Alcohol | Ethanol | 5.0 | 5.0 |
| Moisturize r | Glycerin | 1.0 | 1.0 |
| Thickener | disteardimonium hectorite | 0.5 | 0.5 |
| Activator | PEG-9 polydimethylsiloxyethyl dimethicone | 2.0 | 2.0 |
| Dispersant | Isostearic acid | 0.5 | 0.5 |
| Oil content | Diisopropyl sebacate | 10 | 10 |
| | Triethylhexanoin | 10 | 10 |
| | Isododecane | 10 | 10 |
| | Dimethicone | 10 | 10 |
| First compound | Compound I | 1.0 | - |
| Second compound | Octocrylene (molecular weight: 361) | - | 1.0 |
| UV scattering agent | Aluminum stearate-treated titanium oxide | 2.0 | 2.0 |
| | Triethoxycaprylylsilane-treated zinc oxide | 5.0 | 5.0 |
| Usability powder | Silica powder | 5.0 | 5.0 |
| Preservati ve | Phenoxyethanol | 0.5 | 0.5 |
| Buffer | Sodium chloride | 0.1 | 0.1 |
| Chelating agent | EDTA-3Na | 0.2 | 0.2 |
| Total | | 100 | 100 |
| Evaluation | Integrated value of absorbance | Same degree as Reference Example 2 | Same degree as Reference Example 1 |

As shown in Table 4, when the ultraviolet absorption abilities of the composition of Reference Example 1 and the composition of Reference Example 2 were compared, it was found that they were at the same level. From this, it is understood that in these compositions, the ultraviolet absorption ability of the first compound and the ultraviolet absorption ability of the second compound are comparable.

### <<Preparation of Oil-Based Cosmetic Compositions

### <Examples 12 to 20 and Comparative Examples 14 to 24>

The compositions of Examples 12 to 20 and Comparative Examples 14 to 24 were prepared based on the compositions (% by mass) of Tables 5 and 6 below. Note that the compositions of Comparative Examples 15 to 23, which did not contain the "first compound" and were compensated with an increased amount of the oil content, were otherwise the same as the compositions of Examples 12 to 20, respectively. Furthermore, the composition of Comparative Example 24, which did not contain the "first compound" and was compensated with an increased amount of the oil content, was otherwise the same as the composition of Comparative Example 14.

### (Evaluation)

In the same manner as Example 1 described above, the integrated value of absorbance at 290 to 400 nm was determined for each composition prepared, and the ultraviolet absorption ability improvement effect of each of the compositions of Examples 12 to 20 and Comparative Example 14 was determined as the "boost rate." The results are shown in Table 5.

Note that when calculating the boost rate, Comparative Examples 15 to 24 were used as references for Examples 12 to 20 and Comparative Example 14, respectively.

**[Table 5]**

| (Table 5: Example 12 to 20 and Comparative Example 14) | | | | | | | | | | | |
|---|---|---|---|---|---|---|---|---|---|---|---|
| Composition | | Example 12 | Example 13 | Example 14 | Example 15 | Example 16 | Example 17 | Example 18 | Example 19 | Example 20 | Comparative Example 14 |
| Oil content | Diisopropyl sebacate | 33 | 33 | 33 | 33 | 35 | 35 | 36 | 33 | 35 | 36 |
| | Hydrogenated polydecene | 20 | 20 | 20 | 20 | 20 | 20 | 20 | 20 | 20 | 20 |
| | Diphenylsiloxyphenyl trimethicone | 20 | 20 | 20 | 20 | 20 | 20 | 20 | 20 | 20 | 20 |
| First compound | Compound I | 1.0 | 1.0 | 1.0 | 1.0 | 1.0 | 1.0 | 1.0 | 1.0 | 1.0 | 1.0 |
| Second compound | Ethylhexyl methoxycinnamate | 5.0 | - | - | - | - | - | - | - | - | - |
| | Octocrylene | - | 5.0 | - | - | - | - | - | - | - | - |
| | Ethylhexyl salicylate | - | - | 5.0 | - | - | - | - | - | - | - |
| | Homosalate | - | - | - | 5.0 | - | - | - | - | - | - |
| | Bisethylhexyloxyphenol methoxyphenyltriazine | - | - | - | - | 3.0 | - | - | - | - | - |
| | Diethylaminohydroxybenzoylhexyl benzoate | - | - | - | - | - | 3.0 | - | - | - | - |
| | Ethylhexyltriazone | - | - | - | - | - | - | 2.0 | - | - | - |
| | Polysilicone-15 | - | - | - | - | - | - | - | 5.0 | - | - |
| | t-butylmethoxydibenzoylmethane | - | - | - | - | - | - | - | - | 3.0 | - |
| Third compound | Oxybenzone-3 | - | - | - | - | - | - | - | - | - | 2.0 |
| Other | Other | 21 | 21 | 21 | 21 | 21 | 21 | 21 | 21 | 21 | 21 |
| Total | | 100 | 100 | 100 | 100 | 100 | 100 | 100 | 100 | 100 | 100 |
| Evaluation | Boost rate | 122% | 132% | 140% | 135% | 141% | 143% | 150% | 149% | 142% | 111% |
| | Boost rate calculation reference | Comparative Ex ample 15 | Comparative Example 16 | Comparative Ex ample 17 | Comparative Ex ample 18 | Comparative Ex ample 19 | Comparative Ex ample 20 | Comparative Ex ample 21 | Comparative Ex ample 22 | Comparative Ex ample 23 | Comparative Ex ample 24 |

**[Table 6]**

| (Table 6: Comparative Example 15 to 24) | | | | | | | | | | | |
|---|---|---|---|---|---|---|---|---|---|---|---|
| Composition | | Comparative Example 15 | Comparative Example 16 | Comparative Example 17 | Comparative Example 18 | Comparative Example 19 | Comparative Example 20 | Comparative Example 21 | Comparative Example 22 | Comparative Example 23 | Comparative Example 24 |
| Oil content | Diisopropyl sebacate | 34 | 34 | 34 | 34 | 36 | 36 | 37 | 34 | 36 | 37 |
| | Hydrogenated polydecene | 20 | 20 | 20 | 20 | 20 | 20 | 20 | 20 | 20 | 20 |
| | Diphenylsiloxyphenyl trimethicone | 20 | 20 | 20 | 20 | 20 | 20 | 20 | 20 | 20 | 20 |
| First compound | Compound I | - | - | - | - | - | - | - | - | - | - |
| Second compound | Ethylhexyl methoxycinnamate | 5.0 | - | - | - | - | - | - | - | - | - |
| | Octocrylene | - | 5.0 | - | - | - | - | - | - | - | - |
| | Ethylhexyl salicylate | - | - | 5.0 | - | - | - | - | - | - | - |
| | Homosalate | - | - | - | 5.0 | - | - | - | - | - | - |
| | Bisethylhexyloxyphenol methoxyphenyltriazine | - | - | - | - | 3.0 | - | - | - | - | - |
| | Diethylaminohydroxybenzoylhexyl benzoate | - | - | - | - | - | 3.0 | - | - | - | - |
| | Ethylhexyltriazone | - | - | - | - | - | - | 2.0 | - | - | - |
| | Polysilicone-15 | - | - | - | - | - | - | - | 5.0 | - | - |
| | t-butylmethoxydibenzoylmethane | - | - | - | - | - | - | - | - | 3.0 | - |
| Third compound | Oxybenzone-3 | - | - | - | - | - | - | - | - | - | 2.0 |
| Other | Other | 21 | 21 | 21 | 21 | 21 | 21 | 21 | 21 | 21 | 21 |
| Total | | 100 | 100 | 100 | 100 | 100 | 100 | 100 | 100 | 100 | 100 |

The details of "other" components of Tables 5 and 6 are as follows:

| | |
|---|---|
| ·Ethanol | 5.0% by mass |
| ·Glycerin | 1.0% by mass |
| ·Hydroxystearic acid | 6.0% by mass |
| ·Polyamide-8, pentaerythrityl tetra(di-t-butylhydroxyhydrocinnamate) | 2.0% by mass |
| ·Dibutyl lauroyl glutamide | 2.0% by mass |
| ·Silica powder | 5.0% by mass |

As is clear from Table 5, Examples 12 to 20, which contained the first and second compounds, all had large boost rates (i.e., the ultraviolet absorption ability in the UVA and UVB regions was greatly improved) as compared to Comparative Examples 15 to 23, which contained, among the first to third compounds, only the second compound. From this, it is understood that the combination of the first compound and the second compound produces a synergistic effect.

Comparative Example 14, which contained the first and third compounds, had a small boost rate (i.e., there was no significant improvement in ultraviolet absorption ability in the UVA and UVB regions) as compared to Comparative Example 24, which contained, among the first to third compounds, only the third compound. From this, it is understood that no synergistic effect is produced by the combination of the first compound and the third compound.

### <<Preparation of Oil-in-Water Cosmetic Compositions

### <Examples 21 to 31 and Comparative Examples 25 to 37>

The compositions of Examples 21 to 31 and Comparative Examples 25 to 37 were prepared based on the compositions of Tables 7 and 8 below. Note that the compositions of Comparative Examples 26 to 36, which did not contain the "first compound" and were compensated with an increased amount of water, were otherwise the same as the composition of Examples 21 to 31, respectively. Furthermore, the composition of Comparative Example 37, which did not contain the "first compound" and was compensated with an increased amount of water, was otherwise the same as the composition of Comparative Example 25.

### (Evaluation)

In the same manner as Example 1 above, the integrated value of absorbance at 290 to 400 nm was determined for each prepared composition, and the ultraviolet absorption ability improvement effect of each of the compositions of Examples 21 to 31 and Comparative Example 25 was determined as the "boost rate." The results are shown in Table 7.

Note that when calculating the boost rate, Comparative Examples 26 to 37 were used as references for Examples 21 to 31 and Comparative Example 25, respectively.

**[Table 7]**

| (Table 7: Example 21 to 31 and Comparative Example 25) | | | | | | | | | | | | | |
|---|---|---|---|---|---|---|---|---|---|---|---|---|---|
| Composition | | Example 21 | Example 22 | Example 23 | Example 24 | Example 25 | Example 26 | Example 27 | Example 28 | Example 29 | Example 30 | Example 31 | Comparativ e Example 25 |
| Water | Water | Balance | Balance | Balance | Balance | Balance | Balance | Balance | Balance | Balance | Balance | Balance | Balance |
| First compound | Compound I | 1.0 | 1.0 | 1.0 | 1.0 | 1.0 | 1.0 | 1.0 | 1.0 | 1.0 | 1.0 | 1.0 | 1.0 |
| Second compound | Ethylhexyl methoxycinnamate | 5.0 | - | - | - | - | - | - | - | - | - | - | - |
| | Octocrylene | - | 5.0 | - | - | - | - | - | - | - | - | - | - |
| | Ethylhexyl salicylate ATE | - | - | 5.0 | - | - | - | - | - | - | - | - | - |
| | Homosalate | - | - | - | 5.0 | - | - | - | - | - | - | - | - |
| | Bisethylhexyloxyphenol methoxyphenyltriazine | - | - | - | - | 3.0 | - | - | - | - | - | - | - |
| | Diethylaminohydroxybenzoylhex yl benzoate | - | - | - | - | - | 3.0 | - | - | - | - | - | - |
| | Ethylhexyltriazone | - | - | - | - | - | - | 2.0 | - | - | - | - | - |
| | Polysilicone-15 | - | - | - | - | - | - | - | 5.0 | - | - | - | - |
| | t-butylmethoxydibenzoylmethane | - | - | - | - | - | - | - | - | 3.0 | - | - | - |
| | Phenylbenzimidazole sulfonic acid | - | - | - | - | - | - | - | - | - | 2.0 | - | - |
| | Terephthalylidene dicamphor sulfonic acid | - | - | - | - | - | - | - | - | - | - | 2.0 | - |
| Third compound | Oxybenzone-3 | - | - | - | - | - | - | - | - | - | - | - | 2.0 |
| Buffer | Buffer | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount |
| Preservative | Phenoxyethanol | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount |
| Other | Other | 52.82 | 52.82 | 52.82 | 52.82 | 52.82 | 52.82 | 52.82 | 52.82 | 52.82 | 52.82 | 52.82 | 52.82 |
| Total | | 100 | 100 | 100 | 100 | 100 | 100 | 100 | 100 | 100 | 100 | 100 | 100 |
| Evaluation | Boost rate | 121% | 131% | 133% | 125% | 136% | 139% | 142% | 141% | 135% | 138% | 129% | 112% |
| | Boost rate calculation reference | Comp Ex 26 | Comp Ex 27 | Comp Ex 28 | Comp Ex 29 | Comp Ex 30 | Comp Ex 31 | Comp Ex 32 | Comp Ex 33 | Comp Ex 34 | Comp Ex 35 | Comp Ex 36 | Comp Ex 37 |

**[Table 8]**

| (Table 8: Comparative Example 26 to 37) | | | | | | | | | | | | | |
|---|---|---|---|---|---|---|---|---|---|---|---|---|---|
| Composition | | Comparativ e Example 26 | Comparativ e Example 27 | Comparativ e Example 28 | Comparativ e Example 29 | Comparativ e Example 30 | Comparativ e Example 31 | Comparativ e Example 32 | Comparativ e Example 33 | Comparativ e Example 34 | Comparativ e Example 35 | Comparativ e Example 36 | Comparativ e Example 37 |
| Water | Water | Balance | Balance | Balance | Balance | Balance | Balance | Balance | Balance | Balance | Balance | Balance | Balance |
| First compound | Compound I | - | - | - | - | - | - | - | - | - | - | - | - |
| Second compound | Ethylhexyl methoxycinnamate | 5.0 | - | - | - | - | - | - | - | - | - | - | - |
| | Octocrylene | - | 5.0 | - | - | - | - | - | - | - | - | - | - |
| | Ethylhexyl salicylate ATE | - | - | 5.0 | - | - | - | - | - | - | - | - | - |
| | Homosalate | - | - | - | 5.0 | - | - | - | - | - | - | - | - |
| | Bisethylhexyloxyphenol methoxyphenyltriazine | - | - | - | - | 3.0 | - | - | - | - | - | - | - |
| | Diethylaminohydroxybenzoylhexyl benzoate | - | - | - | - | - | 3.0 | - | - | - | - | - | - |
| | Ethylhexyltriazone | - | - | - | - | - | - | 2.0 | - | - | - | - | - |
| | Polysilicone-15 | - | - | - | - | - | - | - | 5.0 | - | - | - | - |
| | t-butylmethoxydibenzoylmethane | - | - | - | - | - | - | - | - | 3.0 | - | - | - |
| | Phenylbenzimidazole sulfonic acid | - | - | - | - | - | - | - | - | - | 2.0 | - | - |
| | Terephthalylidene dicamphor sulfonic acid | - | - | - | - | - | - | - | - | - | - | 2.0 | - |
| Third compound | Oxybenzone-3 | - | - | - | - | - | - | - | - | - | - | - | 2.0 |
| Buffer | Buffer | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount |
| Preservative | Phenoxyethanol | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount | Appropriat e amount |
| Other | Other | 52.82 | 52.82 | 52.82 | 52.82 | 52.82 | 52.82 | 52.82 | 52.82 | 52.82 | 52.82 | 52.82 | 52.82 |
| Total | | 100 | 100 | 100 | 100 | 100 | 100 | 100 | 100 | 100 | 100 | 100 | 100 |

The details of "other" components of Tables 7 and 8 are as follows:

| | |
|---|---|
| ·Glycerin | 4.0% by mass |
| ·1,3-Butylene glycol | 7.0% by mass |
| ·Succinoglycan | 0.12% by mass |
| ·Sucrose fatty acid ester | 3.0% by mass |
| ·(Dimethylacrylamide/acryloyldimethyltaurine Na) crosspolymer | 0.6% by mass |
| ·PEG-60 hydrogenated castor oil | 1.6% by mass |
| ·Diisopropyl sebacate | 4.0% by mass |
| ·Non-volatile dimethicone | 2.0% by mass |
| ·PPG-17 | 1.0% by mass |
| ·Cyclomethicone | 12% by mass |
| ·Triethylhexanoin | 5.0% by mass |
| ·Isostearic acid | 1.0% by mass |
| ·Sorbitan sesquiisostearate | 0.5% by mass |
| ·Dimethicone-treated silica-coated fine particle zinc oxide | 10% by mass |
| ·Silica powder | 1.0% by mass |

The details of "buffers" of Tables 7 and 8 are as follows:
Citric acid: appropriate amount
Sodium citrate: appropriate amount
EDTA-3Na: appropriate amount

As is clear from Table 7, Examples 21 to 31, which contained the first and second compounds, all had large boost rates (i.e., the ultraviolet absorption ability in the UVA and UVB regions was greatly improved) as compared to Comparative Examples 26 to 36, which contained, among the first to third compounds, only the second compound. From this, it is understood that the combination of the first compound and the second compound produces a synergistic effect.

Comparative Example 25, which contained the first and third compounds, had a small boost rate (i.e., there was no significant improvement in ultraviolet absorption ability in the UVA and UVB regions) as compared to Comparative Example 37, which contained, among the first to third compounds, only the third compound. From this, it is understood that no synergistic effect is produced by the combination of the first compound and the third compound.

## Claims

1. A cosmetic composition, comprising components (i) and (ii) described below, and an oil content which is capable of at least partially dissolving these components:
(i) a first compound, which has a structure of formula (I) below: 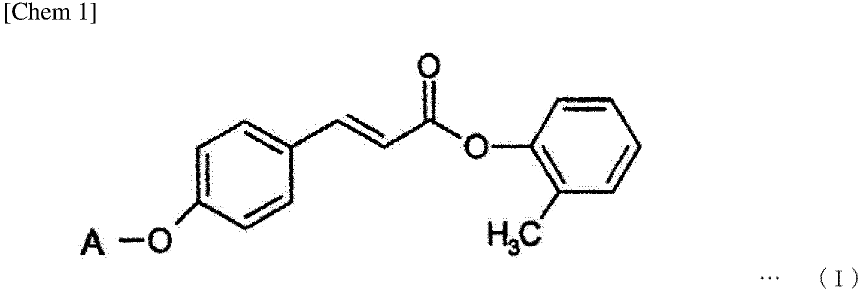 where -OA represents an alkoxy group, and
(ii) a second compound, which is different from the first compound, has a structure of any of (II-1), (II-2), (II-3), and (II-4) described below, and has a molecular weight of 250 or more: 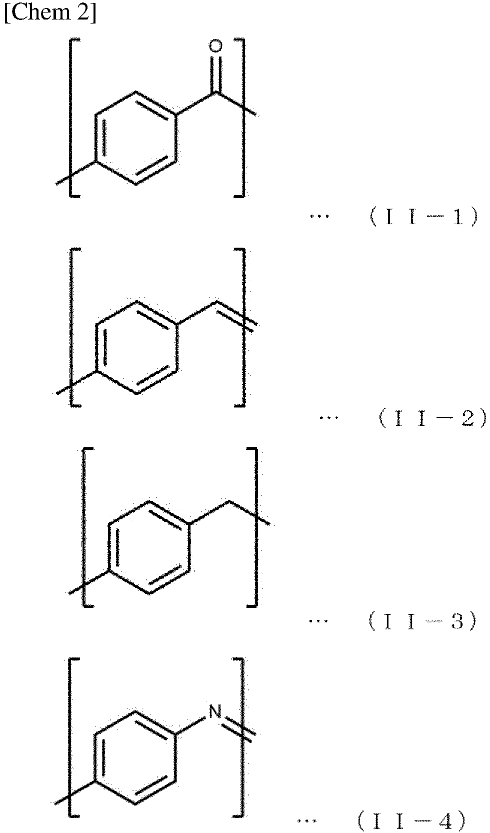 where the benzene ring may have a substituent.

2. The composition according to claim 1, wherein the second compound is at least one compound selected from the group consisting of ethylhexyl methoxycinnamate, octocrylene, ethylhexyl salicylate, homosalate, bisethylhexyloxyphenol methoxyphenyl triazine, diethylaminohydroxybenzoylhexyl benzoate, ethylhexyl triazone, polysilicone-15, t-butylmethoxydibenzoylmethane, phenylbenzimidazole sulfonic acid, and terephthalylidene dicamphor sulfonic acid.

3. The composition according to claim 1 or 2, wherein the oil content contains a polar oil.

4. The composition according to claim 3, wherein the first compound is 0.01 parts by mass or more and 50 parts by mass or less relative to a total of 100 parts by mass of the polar oil and the first compound.

5. The composition according to any one of claims 1 to 4, which is an oil-in-water cosmetic.

6. The composition according to any one of claims 1 to 4, which is a water-in-oil cosmetic.

7. The composition according to any one of claims 1 to 4, which is an oil-based cosmetic.

8. The composition according to any one of claims 1 to 7, which is a sunscreen cosmetic composition.
